(19) Europäisches Patentamt
European Patent Office
Office européen des brevets

(11) Veröffentlichungsnummer: **0 248 187**
**A2**

(12) **EUROPÄISCHE PATENTANMELDUNG**

(21) Anmeldenummer: **87105787.3**

(22) Anmeldetag: **18.04.87**

(51) Int. Cl.⁴: **C07D 491/04**, A61K 31/40, //(C07D491/04,317:00,209:00)

(30) Priorität: **30.04.86 DE 3614758**

(43) Veröffentlichungstag der Anmeldung: **09.12.87 Patentblatt 87/50**

(84) Benannte Vertragsstaaten: **AT BE CH DE ES FR GB IT LI NL SE**

(71) Anmelder: **Merck Patent Gesellschaft mit beschränkter Haftung**
**Frankfurter Strasse 250**
**D-6100 Darmstadt(DE)**

(72) Erfinder: **Böttcher, Henning, Dr.**
**Soderstrasse 95**
**D-6100 Darmstadt(DE)**
Erfinder: **Hausberg, Hans-Heinrich, Dr.**
**Odenwaldstrasse 30**
**D-6105 Ober-Ramstadt(DE)**
Erfinder: **Seyfried, Christoph, Dr.**
**Mathildenstrasse 6**
**D-6104 Seeheim-Jugenheim(DE)**
Erfinder: **Minck, Klaus-Otto, Dr.**
**Büchestrasse 8**
**D-6105 Ober-Ramstadt(DE)**

(54) **Indolderivate.**

(57) 3-[4-(4-Phenyl-1,2,3,6-tetrahydropyridyl)-butyl]-5,6-methylendioxyindol (I) und dessen Salze beeinflusssen das Zentralnervensystem.

EP 0 248 187 A2

## Indolderivat

Gegenstand der Erfindung sind das neue 3-[4-(4-Phenyl-1,2,3,6-tetrahydropyridyl)-butyl]-5,6-methylendioxyindol (I) und dessen Salze.

Der Erfindung lag die Aufgabe zugrunde, neue Verbindungen aufzufinden, die zur Herstellung von Arzneimitteln verwendet werden können.

Es wurde gefunden, daß die genannten Substanzen bei guter Verträglichkeit wertvolle pharmakologische Eigenschaften besitzen. So zeigen sie z.B. das Zentralnervensystem beeinflussende, vorzugsweise dämpfende (z.B. sedierende, tranquillierende, neuroleptische und/oder antidepressive) Wirkungen. Im einzelnen haben die Verbindungen eine dämpfende Wirkung auf das Verhalten bei Mäusen (Methodik vgl. Irwin, Psychopharmacologia 13(1968), 222-257), hemmen bei Mäusen das durch Apomorphin induzierte Kletterverhalten (Methodik vgl. Costall et al., European J. Pharmacol. 50 (1968), 39-50) oder induzieren contralaterales Drehverhalten in Hemiparkinson-Ratten (feststellbar nach der Methode von Ungerstedt et al., Brain Res. 24 (1970), 485-493), ohne daß nennenswerte kataleptische Nebenwirkungen auftreten (Methodik vgl. Dolini-Stola, Pharmakopsychiat. 6 (1973), 189-197). Weiterhin hemmen die Substanzen die Bindung von tritiierten Dopaminagonisten und -antagonisten an striäre Rezeptoren (feststellbar nach der Methode von Schwarcz et al., J. Neurochemistry 34 (1980), 772-778, und Creese et al., European J.Pharmacol. 46 - (1977), 377-381). Zusätzlich hemmen die Verbindungen den Zungen-Kieferreflex bei der narkotisierten Ratte (feststellbar in Anlehnung an die Methoden von Barnett et. al., European J.Pharmacol. 21 (1973), 178-182, und von Ilhan et al., European J.Pharmacol. 33 (1975), 61-64). Weiterhin treten analgetische und blutdrucksenkende Wirkungen auf; so wird der bei kathetertragenden wachen, spontan hypertonen Ratten (Stamm SHR/NIH-MO//CHB-EMD; Methode vgl. Weeks und Jones, Proc.Soc.Exptl.Biol.Med. 104 (1960), 646-648) direkt gemessene arterielle Blutdruck nach intragastraler Gabe der Verbindungen gesenkt.

Die Verbindung I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung ist ferner ein Verfahren zur Herstellung der Verbindung (I) sowie von ihren Salzen, dadurch gekennzeichnet, daß man ein 3-(4-$X^1$-butyl)-5,6-methylendioxyindol (II),

worin

$X^1$ X oder $NH_2$ und

X Cl, Br, J, OH oder eine reaktionsfähige funktionelle abgewandelte OH-Gruppe bedeuten

mit einer Verbindung der Formel III

$X^2\text{-}CH_2CH_2\text{-}C(C_6H_5)=CH\text{-}CH_2X^3$ III

worin

$X^2$ und $X^3$ gleich oder verschieden sein können und, falls $X^1 = NH_2$ ist, jeweils X, andernfalls zusammen NH bedeuten

umsetzt

oder daß man eine sonst (I) entsprechende Verbindung, die jedoch an Stelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt

oder daß man eine sonst (I) entsprechende Verbindung, die jedoch an Stelle eines oder mehrerer Wasserstoffatome eine oder mehrere solvolysierbare Gruppe(n) enthält, mit einem solvolysierenden Mittel behandelt

oder daß man ein 3-[4-(3,4-Di-E-4-phenylpiperidino)-butyl]-5,6-methylendioxyindol (IV)

worin

der eine Rest E X, CN oder $NH_2$,

der andere Rest E H bedeutet und

X die angegebene Bedeutung hat

mit einem HE-abspaltendem Mittel behandelt

und/oder daß man gegebenenfalls die Base (I) durch Behandeln mit einer Säure in eines ihrer Salze umwandelt.

Die Herstellung der Verbindung I erfolgt im übrigen nach an sich bekannten Methoden, wie sie in der Literatur (z.B. in der DE-OS 28 27 874) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Verbindungen der Formel II ist $X^1$ vorzugsweise X; dementsprechend sind in den Verbindungen der Formel III $X^2$ und $X^3$ vorzugsweise zusammen NH. Der Rest X ist vorzugsweise Cl oder Br; er kann jedoch auch J, OH oder eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1-oder 2-Naphthalin-sulfonyloxy).

Dementsprechend sind die Verbindungen der Formel I insbesondere durch Umsetzung von 3-(4-Chlorbutyl)-oder 3-(4-Brombutyl)-5,6-methylendioxyindol mit 4-Phenyl-1,2,3,6-tetrahydropyridin (IIIa) erhältlich.

Die Verbindungen der Formeln II und III sind zum Teil bekannt; die nicht bekannten Verbindungen der Formeln II und III können leicht analog zu den bekannten Verbindungen herstellt werden. 3-(4-Hydroxybutyl)-5,6-methylendioxyindol (IIa) ist z.B. durch Reduktion der entsprechenden Carbonsäure oder ihrer Ester erhältlich. Behandeln mit Thionylchlorid, Bromwasserstoff, Phosphortribromid oder ähnlichen Halogenverbindungen liefert die entsprechenden Halogenide. Die entsprechenden Sulfonyloxyverbindungen sind erhältlich aus IIa durch Umsetzung mit den entsprechenden Sulfonsäurechloriden. 3-(4-Jodbutyl)-5,6-methylendioxyindol ist z.B. durch Einwirkung von Kaliumjodid auf den zugehörigen p-Toluolsulfonsäureester erhältlich. 3-(4-Aminobutyl)-5,6-methylendioxyindol ist z.B. aus den Halogeniden mit Phthalimidkalium oder durch Reduktion des entsprechenden Nitrils herstellbar.

Die Verbindungen der Formel III sind weitgehend bekannt (vgl. DE-OS 20 60 816).

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali-oder Erdalkalimetalhydroxids, -carbonats oder -bicarbonats oder eines anderen Salzes einer schwachen Säure der Alkali-oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Ver bindung IIIa kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Es ist ferner möglich, die Verbindung (I) zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B.p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr dieser Gruppen bzw. zusätzlichen Bindungen enthalten, reduktiv in (I) überzuführen. Vorzugsweise bedient man sich hierzu des nascierenden Wasserstoffs oder komplexer Metallhydride, ferner der Reduktion nach Wolff-Kishner.

Bevorzugte Ausgangsstoffe für die Reduktion entsprechen der Formel V

Ind-L-Z V

worin

Ind die 5,6-Methylendioxy-3-indolyl-gruppe,

L $-(CH_2)_4$-oder eine der Butylkette entsprechende Kette bedeutet, worin jedoch eine oder mehrere $-CH_2$-Gruppe(n) durch -CO-Gruppe(n) und/oder ein oder mehrere Wasserstoffatom(e) durch OH-Gruppe(n) ersetzt sind und

Z 4-Phenyl-1,2,3,6-tetrahydropyridyl oder

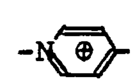

$C_6H_5$ $An^{\theta}$ und

$An^{\theta}$ ein Anion bedeutet,
worin aber nicht gleichzeitig Z = 4-Phenyl-1,2,3,6-tetrahydropyridyl und L = $-(CH_2)_4$-sein können.

In den Verbindungen der Formel V ist L bevorzugt $-(CH_2)_3-CO-$, $-(CH_2)_2-CO-CH_2-$, $-CH_2-CO-(CH_2)_2-$, $-CO-(CH_2)_3-$, $-CO-(CH_2)_2-CO-$, $-(CH_2)_3-CHOH-$, $-CO-(CH_2)_2-CHOH-$, $-CHOH-(CH_2)_2-CO-$.

Verbindungen der Formel V sind z.B. herstellbar durch Umsetzung von IIIa oder 4-Phenylpyridin mit einer Verbindung der Formel VI
Ind-L-$X^1$ VI
worin
Ind, L und $X^1$ die oben angegebenen Bedeutungen haben,
unter den Bedingungen, die oben für die Umsetzung von II und III angegeben sind.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl-oder Isoamylalkohol oder Phenol. Man kann ferner eine Aluminium-Nickel-Legierung in alkalischwäßriger Lösung, gegenbenenfalls unter Zusatz von Ethanol verwenden. Auch Natrium-oder Aluminiumamalgam in wäßrigalkoholischer oder wäßriger Lösung sind zur Erzeugung des nascierenden Wasserstoffs geeignet. Die Umsetzung kann auch in heterogener Phase durchgeführt werden, wobei man zweckmäßig eine wäßrige und eine Benzol-oder Toluol-Phase verwendet.

Als Reduktionsmittel können ferner besonders vorteilhaft komplexe Metallhydride, wie $LiAlH_4$, $NaBH_4$, Diisobutylaluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit $NaBH_4$ sind in erster Linie Alkohole wie Methanol oder Ethanol, ferner Wasser sowie wäßrige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden (z.B. solchen der Formel V, worin L -- $(CH_2)_3$-CO-bedeutet) mit $LiAlH_4$ in THF bei Temperaturen zwischen etwa 0 und 66° zu $CH_2$-Gruppen reduzieren. Eine Reduktion der Pyridiniumsalze der Formel V (worin An vorzugsweise Cl oder Br bedeutet) kann z.B. mit $NaBH_4$ in Wasser, Methanol oder Ethanol, falls erwünscht unter Zusatz einer Base wie NaOH bei Temperaturen zwischen etwa 0 und 80° erfolgen.

Es ist ferner möglich, eine oder mehrere Carbonylgruppen nach der Methode von Wolff-Kishner zu $CH_2$-Gruppen zu reduzieren, z.B. durch Behandlung mit wasserfreiem Hydrazin in absolutem Ethanol unter Druck bei Temperaturen zwischen etwa 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3-4 Stunden gekocht. Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Verbindungen, die sonst (I) entsprechen, aber an Stelle eines oder mehrerer H-Atome eine oder mehrere solvolysierbare Gruppe(n) enthalten, können zu der Verbindung (I) solvolysiert, insbesondere hydrolysiert werden.

Die Ausgangsstoffe für die Solvolyse sind beispielsweise erhältlich durch Reaktion von IIIa mit Verbindungen, die der Formel II ($X^1$ = X) entsprechen, aber an Stelle eines oder mehrerer H-Atome eine oder mehrere solvolysierbare Gruppe(n) enthalten. So können insbesondere 1-Acylindolderivate (entsprechend (I), aber in 1-Stellung des Indolrests eine Acylgruppe enthaltend, vorzugsweise eine Alkanoyl-, Alkylsulfonyl-oder Arylsulfonylgruppe mit jeweils bis zu 10 C-Atomen, wie Methan-, Benzol-oder p-Toluolsulfonyl) zu den entsprechenden in der 1-Stellung des Indolringes unsubstituierten Indolderivaten hydrolysiert werden, z.B. in saurem, besser in neutralem oder alkalischem Medium bei Temperaturen zwischen 0 und 200°.

Als basische Katalysatoren verwendet man zweckmäßig Natrium-, Kalium-oder Calciumhydroxid, Natrium-oder Kaliumcarbonat, oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonderes die Wasser enthaltenden Gemische. Eine Hydrolyse kann auch bereits beim Behandeln mit Wasser allein erfolgen, insbesondere in der Siedehitze.

Man gelangt ferner zu (I), indem man aus Verbindungen der Formel IV unter Ausbildung einer Doppelbindung HE abspaltet. Entsprechend der Definition von E kann es sich z.B. handeln um eine Abspaltung von Halogenwasserstoff, Wasser (Dehydratisierung), einer Carbonsäure oder einer anderen Säure, von Ammoniak oder von HCN. Die Ausgangsstoffe der Formel IV sind z.B. erhältlich durch Umsetzung von II ($X^1$ = X) mit einer Verbindung der Formel VII

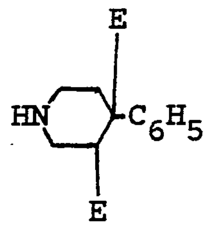

VII

worin E die angegebene Bedeutung hat.

Falls einer der Reste E = Hal ist, kann dieser Substituent unter basischen Reaktionsbedingungen leicht eliminiert werden. Als Basen können verwendet werden: Alkalimetallhydroxide, Alkalimetallcarbonate, Alkoholate, wie z.B. Kalium-tert.-butylat, Amine, wie z.B. Dimethylanilin, Pyridin, Collidin oder Chinolin; als Lösungsmittel benutzt man z.B. Benzol, Toluol, Cyclohexan, THF oder tert.-Butanol. Die als Basen verwendeten Amine können auch im Überschuß als Lösungsmittel eingesetzt werden. Bedeutet der eine der Reste E eine OH-Gruppe, so benutzt man als wasserabspaltende Mittel vorzugsweise Säuren wie Essigsäure, Salzsäure oder Gemische beider. Der Zusatz eines Lösungsmittels (z.B. Wasser oder Ethanol) kann von Vorteil sein. Die Eliminierung von Acyl-, Alkylsulfonyl-sowie Alkoxysulfonyl-oxy-oder Amino-Resten kann unter ähnlichen Bedingungen durchgeführt werden. Eine Eliminierung von Sulfonsäure-Resten, z.B. die der Mesylate oder Tosylate, erfolgt schonend durch Kochen in DMF oder Dimethylsulfoxid mit Alkalimetallcarbonaten, z.B. $Li_2CO_3$, oder mit Kaliumacetat. Ammoniak kann bereits durch Erhitzen der Salze der entsprechenden Aminoverbindungen (insbesondere der 4-Aminoderivate) abgespalten werden. In ähnlicher Weise kann HCN aus Verbindungen der Formel IV (eine Gruppe E = CN) durch Erhitzen abgespalten werden. Die Eliminierung von HE aus IV erfolgt allgemein bei Temperaturen zwischen 0 und etwa 250°, vorzugsweise zwischen 50 und 200°.

Eine erhaltene Base (I) kann mit einer Säure in das zugehörige Säureadditionssalz übergeführt werden. Für diese Umsetzung eignen sich bevorzugt Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäuren, Salpetersäure, Sulfaminsäure, ferner organische Säuren, im einzelnen aliphatische, alicyclische, araliphatische, aromatische oder heterocyclische ein-oder mehrbasige Carbon-, Sulfon-oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan-oder Ethansulfonsäure, Ethandisulfonsäure, 2-Hydroxyethansulfonsäure, Benzolsulfonsäure, p-Toluolsulfonsäure, Naphthalin-mono-und disulfonsäuren, Laurylschwefelsäure. Säureadditionssalze, die nicht physiologisch unbedenklich sind (z.B. Pikrate), können sich zur Isolierung und Aufreinigung der Base (I) eignen.

Die freie Base (I) kann, falls gewünscht, aus ihren Salzen durch Behandlung mit starken Basen wie Natrium-oder Kaliumhydroxid, Natrium-oder Kaliumcarbonat in Freiheit gesetzt werden.

Gegenstand der Erfindung ist ferner die Verwendung der Verbindung (I) und ihrer physiologisch unbedenklichen Salze zur Herstellung pharmazeutischer Zubereitungen, insbesondere auf nicht-chemischem Wege. Hierbei können sie zusammen mit mindestens einem Träger-oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend die Verbindung (I) und/oder eines ihrer physiologisch unbedenklichen Salze. Diese Zubereitungen können als Arzneimittel in der Human-oder Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe,

Säfte, Tropfen oder Suppositorien, zur parenteralen Applikation Lösungen, vorzugsweise ölige oder wäßrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs-und/oder Netzmittel, Emulgatoren, Salze zur Beeinflussung des osmotischen Druckes, Puffersubstanzen, Farb-, Geschmacks-und/oder Aromastoffe enthalten. Sie können, falls erwünscht, auch einen oder mehrere weitere Wirkstoffe enthalten, z.B. ein oder mehrere Vitamine.

Gegenstand der Erfindung ist ferner die Verwendung der Verbindung (I) und ihrer physiologisch unbedenklichen Salze bei der therapeutischen Behandlung des menschlichen oder tierischen Körpers und bei der Bekämpfung von Krankheiten, insbesondere von Schizophrenie und psychoreaktiven Störungen und Psychopathien, von Depressionen, von schweren chronischen Schmerzen und von Krankheiten, die mit erhöhtem Blutdruck einhergehen.

Weiterhin können die Verbindungen bei der Behandlung extrapyramidaler Störungen Anwendung finden.

Dabei werden die erfindungsgemäßen Substanzen in der Regel in Analogie zu bekannten, im Handel befindlichen Präparaten (Thioridazin, Haloperidol) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,003 und 10 mg/kg Körpergewicht.

Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfol gungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben.

Beispiel 1

Man rührt eine Lösung von 2,52 g 3-(4-Chlorbutyl)-5,6-methylendioxyindol [oder 2,96 g 3-(4-Brombutyl)-5,6-methylendioxyindol] und 1,6 g IIIa in 10 ml Acetonitril 12 Std. bei 20°, arbeitet wie üblich auf und erhält (I), F. 108-110°, Hydrochlorid, F. 236-238°. Fumarat, F. 153-155°. Maleat, F. 163-165°. Malonat, F. 151°. Succinat, F. 143-144°. Sulfat, F. 233-235°.

Beispiel 2

Ein Gemisch von 4,85 g 3-(4-p-Toluolsulfonyloxy-butyl)-5,6-methylendioxyindol und 3,18 g IIIa wird auf 130° erhitzt. Nach Abklingen der exothermen Reaktion und Erkalten arbeitet man wie üblich auf und erhält (I), F. 108-110°.

Beispiel 3

Man kocht 3,43 g 3-(4-Jodbutyl)-5,6-methylendioxyindol, 1,59 g IIIa und 1,5 g wasserfreies Kaliumcarbonat in 25 ml n-Butanol 2 Std. unter Rühren, läßt erkalten, arbeitet wie üblich auf und erhält (I), F. 108-110°.

Beispiel 4

Ein Gemisch von 2,32 g 3-(4-Aminobutyl)-5,6-methylendioxyindol (erhältlich durch Reaktion von 3-(4-Brombutyl)-5,6-methylendioxyindol mit Phthalimidkalium und anschließende Hydrolyse) und 2,15 g 1,5-Dichlor-3-phenyl-2-penten (vgl. DE-OS 28 27 874) in 40 ml Aceton und 40 ml Wasser wird 24 Std. gekocht und wie üblich aufgearbeitet. Man erhält (I), F. 108-110°.

Beispiel 5

a) Zu einer Lösung von 2,47 g 4-(5,6-Methylendioxy-3-indolyl)-buttersäure in 10 ml THF gibt man 1,62 g N,N-Carbonyldiimidazol, rührt 1 Std. bei 20° und fügt dann eine Lösung von 1,59 g IIIa in 50 ml THF hinzu. Man rührt 1,5 Std. bei 20°, arbeitet wie üblich auf und erhält 3-[4-Oxo-4-(4-phenyl-1,2,3,6-tetrahydropyridyl)-butyl]-5,6-methylendioxyindol, F. 161-163°,

b) Zu einer Suspension von 0,38 g $LiAlH_4$ in 10 ml THF tropft man eine Lösung von 3,88 g der nach a) erhaltenen Verbindung in 10 ml THF unter Rühren. Nach Abklingen der Reaktion gibt man 5 ml Ethylacetat hinzu, arbeitet wie üblich auf und erhält (I), F. 108-110°,

Beispiel 6

Zu einer Lösung von 4,51 g 1-[4-(5,6-Methylendioxy-3-indolyl)-butyl]-4-phenylpyridiniumbromid [erhältlich aus 3-(4-Brombutyl)-5,6-methylendioxyindol und 4-Phenylpyridin] in 50 ml 1 n NaOH gibt man unter Rühren 1 g $NaBH_4$ in 20 ml Wasser und rührt danach noch 3 Std. bei 60°. Nach üblicher Aufarbeitung erhält man (I), F. 108-110°.

Beispiel 7

Man kocht 1 g 1-Benzolsulfonyl-3-[4-(4-phenyl-1,2,3,6-tetrahydropyridyl)-butyl]-5,6-methylendioxyindol [erhältlich aus 1-Benzolsulfonyl-3-(4-chlorbutyl)-5,6-methylendioxyindol und IIIa] mit 0,2 g KOH in 1,5 ml Wasser und 3 ml Ethanol 16 Std., dampft weitgehend ein, arbeitet wie üblich auf und erhält (I), F. 108-110°.

Beispiel 8

Man erhitzt 3,76 g 3-[4-(4-Hydroxy-4-phenyl-1-piperidyl)-butyl]-5,6-methylendioxyindol mit 40 ml 1 n Salzsäure 2 Stunden auf 50°, arbeitet wie üblich auf und erhält (I), F. 108-110°.

Die nachstehenden Beispiele betreffen pharmazeutische Zubereitungen, die Amine der Formel I oder ihre Säureadditionssalze enthalten:

Beispiel A: Tabletten

Ein Gemisch von 1 kg (I)-Hydrochlorid, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

2 kg (I)-Hydrochlorid werden in üblicher Weise in Hartgelatinekapseln gefüllt, so daß jede Kapsel 20 mg des Wirkstoffs enthält.

Beispiel D: Ampullen

Eine Lösung von 1 kg (I)-Hydrochlorid in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die (I) und/oder ein oder mehrere der übrigen physiologisch unbedenklichen Säureadditionssalze von (I) enthalten.

## Ansprüche

1. 3-[4-(4-Phenyl-1,2,3,6-tetrahydropyridyl)-butyl]-5,6-methylendioxyindol (I) und dessen Salze.

2. Verfahren zur Herstellung der Verbindung (I) sowie von ihren Salzen, dadurch gekennzeichnet, daß man ein 3-(4-$X^1$-butyl)-5,6-methylendioxyindol (II),
worin
$X^1$ X oder $NH_2$ und
X Cl, Br, J, OH oder eine reaktionsfähige funktionelle abgewandelte OH-Gruppe bedeuten
mit einer Verbindung der Formel III
$X^2$-$CH_2CH_2$-$C(C_6H_5)$ = CH-$CH_2X^3$ III
worin
$X^2$ und $X^3$ gleich oder verschieden sein können und, falls $X^1$ = $NH_2$ ist, jeweils X, andernfalls zusammen NH bedeuten
umsetzt
oder daß man eine sonst (I) entsprechende Verbindung, die jedoch an Stelle eines oder mehrerer Wasserstoffatome eine oder mehrere reduzierbare Gruppe(n) und/oder eine oder mehrere zusätzliche C-C- und/oder C-B-Bindung(en) enthält, mit einem reduzierenden Mittel behandelt
oder daß man eine sonst (I) entsprechende Verbindung, die jedoch an Stelle eines oder mehrerer Wasserstoffatome eine oder mehrere solvolysierbare Gruppe(n) enthält, mit einem solvolysierenden Mittel behandelt
oder daß man ein 3-[4-(3,4-Di-E-4-phenylpiperidino)-butyl]-5,6-methylendioxyindol (IV)
worin
der eine Rest E X, CN oder $NH_2$,
der andere Rest E H bedeutet und
X die angegebene Bedeutung hat
mit einem HE-abspaltendem Mittel behandelt
und/oder daß man gegebenenfalls die Base (I) durch Behandeln mit einer Säure in eines ihrer Salze umwandelt.

3. Verfahren zur Herstellung pharmazeutischer Zubereitungen, dadurch gekennzeichnet, daß man die Verbindung (I) und/oder eines ihrer physiologisch unbedenklichen Salze zusammen mit mindestens einem festen, flüssigen oder halbflüssigen Träger-oder Hilfsstoff und gegebenenfalls in Kombination mit einem oder mehreren weiteren Wirkstoff(en) in eine geeignete Dosierungsform bringt.

4. Pharmazeutische Zubereitung, gekennzeichnet durch einen Gehalt an der Verbindung (I) und/oder einem ihrer physiologisch unbedenklichen Salze.

5. Verwendung der Verbindung (I) bei der Bekämpfung von Krankheiten.

6. Verwendung der Verbindung (I) und/oder ihrer physiologisch unbedenklichen Salze zur Herstellung von Arzneimitteln.

7. Verbindungen der Formel II.